(19) Europäisches Patentamt
European Patent Office
Office européen des brevets

(11) EP 3 900 790 A1

(12) EUROPEAN PATENT APPLICATION
published in accordance with Art. 153(4) EPC

(43) Date of publication:
27.10.2021 Bulletin 2021/43

(21) Application number: 19898045.0

(22) Date of filing: 20.12.2019

(51) Int Cl.:
*A61Q 5/06* (2006.01) *A61K 8/40* (2006.01)
*A61K 8/41* (2006.01) *A61K 8/49* (2006.01)

(86) International application number:
PCT/JP2019/049976

(87) International publication number:
WO 2020/130114 (25.06.2020 Gazette 2020/26)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(30) Priority: 20.12.2018 JP 2018238673

(71) Applicant: Kao Corporation
Chuo-ku,
Tokyo 103-8210 (JP)

(72) Inventor: INAGAKI, Kensuke
Wakayama-shi, Wakayama 640-8580 (JP)

(74) Representative: Hoffmann Eitle
Patent- und Rechtsanwälte PartmbB
Arabellastraße 30
81925 München (DE)

# (54) COMPOSITION

(57) The present invention relates to a composition containing (A) a compound represented by the following general formula (1) or a salt thereof:

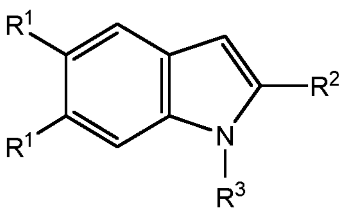

(1)

[two $R^1$'s each independently represent a hydroxy group or an acetoxy group; $R^2$ represents a hydrogen atom or -COOR (R is a hydrogen atom, a methyl group, or an ethyl group); and $R^3$ is a hydrogen atom, an acetyl group, or a methyl or ethyl group]; and (B) a cationic surfactant, wherein the content of the component (B) is 0.25 equivalents or more and less than 2 equivalents relative to the component (A).

EP 3 900 790 A1

## Description

Field of the Invention

The present invention relates to a composition containing a predetermined melanin precursor.

Background of the Invention

Conventionally, air-oxidative hair dye compositions using 5,6-dihydroxyindole, 5,6-dihydroxyindolin, or a derivative thereof, which is a melanin precursor, have been known as a hair dye composition for dyeing gray hair (see, for example, PTLs 1 and 2). Such a melanin precursor is of an air-oxidative type and does not require to use an oxidizing agent, and therefore, on the occasion of being used for a hair dye, it is less in hair damage and high in simplicity as a dye for dyeing hair.

Citation List

Patent Literature

PTL 1: JP 2007-326810 A
PTL 2: JP 2009-137877 A

Summary of the Invention

The present invention relates to a composition containing a predetermined melanin precursor, from which sufficient hair dyeing properties are obtained even with a one-time hair dyeing treatment.
Specifically, the present invention relates to the following [1] and [2].

[1] A composition containing

(A) a compound represented by the following general formula (1) or a salt thereof:

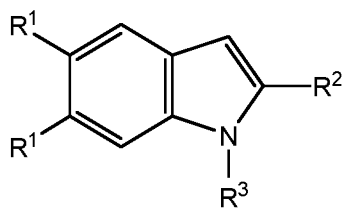

(1)

wherein two $R^{1}$'s each independently represent a hydroxy group or an acetoxy group; $R^2$ represents a hydrogen atom or -COOR (R is a hydrogen atom, a methyl group, or an ethyl group); and $R^3$ is a hydrogen atom, an acetyl group, or a methyl or ethyl group; and
(B) a cationic surfactant,

wherein,
the content of the component (B) is 0.25 equivalents or more and less than 2 equivalents relative to the component (A).
[2] A method for dyeing hair including a step of applying the composition as set forth above in [1] on hair.

Detailed Description of the Invention

[Composition]

The composition of the present invention contains (A) a compound represented by the following general formula (1) or a salt thereof:

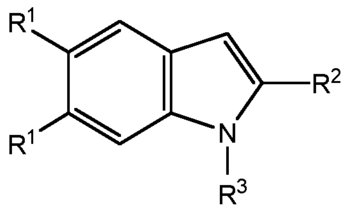

(1)

wherein two $R^1$'s each independently represent a hydroxy group or an acetoxy group; $R^2$ represents a hydrogen atom or -COOR (R is a hydrogen atom, a methyl group, or an ethyl group); and $R^3$ is a hydrogen atom, an acetyl group, or a methyl or ethyl group; and
(B) a cationic surfactant,
wherein,
the content of the component (B) is 0.25 equivalents or more and less than 2 equivalents relative to the component (A).

In view of the fact that the composition of the present invention contains the aforementioned component (A) and a predetermined amount of the component (B), a sufficient hair dyeing effect can be obtained even with a one-time hair dyeing treatment.

The conventional air-oxidative hair dye compositions disclosed in PTLs 1 and 2 are those capable of giving sufficient hair dyeing properties by performing the hair dyeing treatment several times, and it is desired that the hair dyeing properties are more improved.

The present inventor has found that the aforementioned problem can be solved by a composition in which a predetermined melanin precursor and a predetermined amount of a cationic surfactant are used in combination.

When the composition of the present invention is applied on hair, sufficient hair dyeing properties are obtained even with a one-time hair dyeing treatment. The composition of the present invention is, for example, useful as a hair dye.

The reason why the aforementioned effect is obtained by the method of the present invention may be considered as follows.

The component (A) to be used in the present invention is a melanin precursor which penetrates into the hair and is polymerized through air oxidation to produce a melanin pigment, thereby enabling gray hair to be dyed into black hair.

In order to enhance the hair dyeing effect of the composition containing the component (A), there are exemplified (1) suppression of the component (A) from polymerization outside the hair before penetrating into the hair; (2) promotion of penetration of the component (A) into the hair; and (3) promotion of polymerization of the component (A) after penetrating into the hair.

The present inventor paid attention to enhancement of the effect as set forth above in (2) and made extensive and intensive investigations. As a result, it has been found that use of a predetermined amount of a cationic surfactant is effective. In the component (A), the substituent $R^1$ in the general formula (1) has a minus charge. However, it may be considered that this charge is negated using a cationic surfactant, and hydrophobicity is further given, whereby the penetration properties of the component (A) into the hair which is hydrophobic, too are enhanced.

Although the composition of the present invention is not particularly restricted so long as it is one containing the component (A) and a predetermined amount of the compound (B), examples thereof include a hair cleaning agent, such as shampoo, a rinse, a conditioning agent, a treatment agent (inclusive of a non-rinse type), a styling agent, a hair dye, and a hair growth agent. Above these, a hair dye is preferred.

A dosage form of the composition is not particularly resisted, and it is possible to take an arbitrary dosage form, such as a liquid, a foam, a paste, a cream, a solid, and a powder. From the viewpoint of coating properties on hair, a liquid, a paste, or a cream is preferred.

<Component (A)>

The composition of the present invention contains the component (A) that is a compound represented by the following general formula (1) or a salt thereof. The component (A) is a melanin precursor which is polymerized through air oxidation and converted into a melanin polymer (melanin pigment) and acts as a dyeing agent for hair.

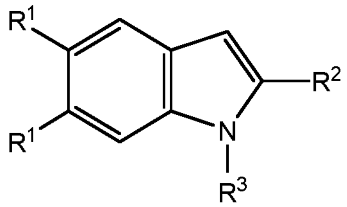

(1)

In the formula, two $R^1$'s each independently represent a hydroxy group or an acetoxy group; $R^2$ represents a hydrogen atom or -COOR (R is a hydrogen atom, a methyl group, or an ethyl group); and $R^3$ is a hydrogen atom, an acetyl group, or a methyl or ethyl group.

The melanin precursor as the component (A) is an indole derivative that is the compound represented by the general formula (1) or a salt thereof, and in the present invention, it can be used alone or in combination of two or more thereof. From the viewpoint of obtaining the effect of the present invention, the component (A) is more preferably an indole derivative.

From the viewpoint of hair dyeing properties and availability, in the general formula (1), $R^1$ is preferably a hydroxy group; $R^2$ is preferably a hydrogen atom or -COOR (R is a hydrogen atom, a methyl group, or an ethyl group), and more preferably a hydrogen atom or -COOH; and $R^3$ is preferably a hydrogen atom.

Examples of the compound represented by the general formula (1) include 5,6-dihydroxyindole, 5,6-dihydroxyindole-2-carboxylic acid, methyl 5,6-dihydroxyindole-2-carboxylate, ethyl 5,6-dihydroxyindole-2-carboxylate, N-methyl-5,6-dihydroxyindole, N-methyl-5,6-dihydroxyindole-2-carboxylic acid, N-ethyl-5,6-dihydroxyindole, N-ethyl-5,6-dihydroxyindole-2-carboxylic acid, N-acetyl-5,6-dihydroxyindole, N-acetyl-5,6-dihydroxyindole-2-carboxylic acid, 5-acetoxy-6-dihydroxyindole, and 5-acetoxy-6-hydroxyindole-2-carboxylic acid.

Examples of the salt of the compound represented by the general formula (1) include a hydrochloride, a hydrobromide, a sulfate, a phosphate, an acetate, a propionate, a lactate, and a citrate of the foregoing compounds. Above all, a hydrobromide is preferred from the viewpoint of availability.

In the general formula (1), when $R^2$ is -COOH, examples of the salt of the compound represented by the general formula (1) include carboxylates thereof ($R^2$ is $-COO^-(X^{n+})_{1/n}$ (n is an integer of 1 or more; and $X^{n+}$ is a cation, such as an alkali metal ion, e.g., $K^+$, $Na^+$, and $Li^+$, an alkaline earth metal ion, e.g., $Ca^{2+}$ and $Mg^{2+}$, and an ammonium ion)). From the viewpoint of solubility of the salt of the compound represented by the general formula (1) in water, the alkali metal ion is preferably $K^+$, $Na^+$, or $Li^+$, more preferably $K^+$ or $Na^+$, and still more preferably $K^+$. In addition, from the same viewpoint, the alkaline earth metal ion is preferably $Ca^{2+}$ or $Mg^{2+}$, and more preferably $Ca^{2+}$.

From the viewpoint of hair dyeing properties, the component (A) is preferably at least one selected from the group consisting of 5,6-dihydroxyindole, 5,6-dihydroxyindole-2-carboxylic acid, and salts thereof, and more preferably at least one selected from the group consisting of 5,6-dihydroxyindole and 5,6-dihydroxyindole-2-carboxylic acid, and still more preferably, the component (A) contains 5,6-dihydroxyindole and 5,6-dihydroxyindole-2-carboxylic acid.

In the case where the component (A) contains 5,6-dihydroxyindole and 5,6-dihydroxyindole-2-carboxylic acid, a molar ratio thereof is preferably in a range of 50/50 to 99.9/0.1, more preferably in a range of 60/40 to 99.8/0.2, still more preferably in a range of 70/30 to 99/1, and yet still more preferably in a range of 80/20 to 95.5.

The molar ratio of 5,6-dihydroxyindole and 5,6-dihydroxyindole-2-carboxylic acid can be quantitatively determined by means of reversed phase HPLC.

From the viewpoint of hair dyeing properties, the content of the component (A) in the composition is preferably 0.05% by mass or more, more preferably 0.1% by mass or more, and still more preferably 0.2% by mass or more, and from the viewpoint of safety and economy, it is preferably 5% by mass or less, more preferably 3% by mass or less, still more preferably 2% by mass or less, and yet still more preferably 1% by mass or less. A specific range of the content of the component (A) in the composition is preferably 0.05 to 5% by mass, more preferably 0.1 to 3% by mass, still more preferably 0.2 to 2% by mass, and yet still more preferably 0.2 to 1% by mass.

<Component (B): Cationic Surfactant>

From the viewpoint of improvement in hair dyeing properties, the composition of the present invention contains a cationic surfactant as the component (B). It may be considered that by using the component (B), penetration properties of the component (A) into the hair are improved due to the aforementioned action mechanism, and the hair dyeing properties are more improved.

Examples of the cationic surfactant include primary to tertiary amine compounds having at least one hydrocarbon group having 8 or more and 22 or less carbon atoms or salts thereof, quaternary ammonium salts, and pyridinium salts.

A counter ion of the quaternary ammonium salt and the pyridinium salt is preferably a halogen ion or an alkyl sulfate ion having 1 to 2 carbon atoms, more preferably a chloride ion, a bromide ion, or a methosulfate ion. The amine

salt is one obtained through neutralization of an amine compound with an inorganic acid or an organic acid.

From the viewpoint of improvement in hair dyeing properties, the total carbon number of the component (B) is preferably 48 or less, more preferably 36 or less, still more preferably 30 or less, yet still more preferably 25 or less, even yet still more preferably 20 or less, and even still more preferably 18 or less. In addition, from the viewpoint of revealing the function as the surfactant, the total carbon number of the component (B) is preferably 11 or more, and more preferably 13 or more. A specific range of the total carbon number of the component (B) is preferably 11 to 48, more preferably 11 to 36, still more preferably 11 to 30, yet still more preferably 11 to 25, even yet still more preferably 11 to 20, even still more preferably 13 to 20, and even still more further preferably 13 to 18.

From the viewpoint of improvement in hair dyeing properties, the component (B) is preferably a quaternary ammonium salt having at least one hydrocarbon group having 8 or more and 22 or less carbon atoms, and more preferably a quaternary ammonium group having at least one hydrocarbon group having 8 or more and 22 or less carbon atoms and having the total carbon number of 36 or less. From the viewpoint of improvement in hair dyeing properties, the quaternary ammonium salt is preferably a mono- or di-long chain alkyl quaternary ammonium salt represented by the following general formula (2).

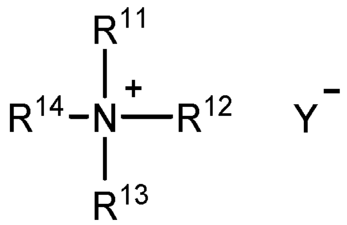
(2)

In the formula, $R^{11}$ represents a linear or branched alkyl group having 8 or more and 22 or less carbon atoms, or a group represented by $R^{15}CONH(CH_2)_m$-, $R^{15}$-O-$(CH_2)_m$-, or $R^{15}COO(CH_2)_m$- ($R^{15}$ represents a linear or branched alkyl group having 8 or more and 22 or less carbon atoms, and m represents a number of 1 or more and 4 or less); $R^{12}$ represents a linear or branched alkyl group having 1 or more and 22 or less carbon atoms, or a group represented by the foregoing $R^{15}CONH(CH_2)_m$-, $R^{15}$-O-$(CH_2)_m$-, or $R^{15}COO(CH_2)_m$-; $R^{13}$ and $R^{14}$ each independently represent an alkyl group having 1 or more and 4 or less carbon atoms; and $Y^-$ represents a chloride ion, a bromide ion, or a methosulfate ion.

In the general formula (2), preferably, $R^{11}$ is a linear or branched alkyl group having 8 or more and 22 or less carbon atoms; $R^{12}$ is a linear or branched alkyl group having 1 or more and 22 or less carbon atoms; $R^{13}$ and $R^{14}$ are each independently an alkyl group having 1 or more and 4 or less carbon atoms; and $Y^-$ is a chloride ion.

The carbon number of the linear or branched alkyl group in $R^{11}$ and $R^{15}$ is 8 or more and 22 or less, preferably 8 or more and 18 or less, more preferably 8 or more and 16 or less, still more preferably 10 or more and 14 or less, and yet still more preferably 12 or more and 14 or less.

The carbon number of the linear or branched alkyl group having 1 or more and 22 or less carbon atoms in $R^{12}$ is preferably 1 or more and 4 or less, and more preferably 1 or more and 3 or less in the case of a mono-long chain alkyl quaternary ammonium salt; and it is preferably 8 or more and 22 or less, more preferably 8 or more and 18 or less, still more preferably 8 or more and 16 or less, yet still more preferably 10 or more and 14 or less, and even yet still more preferably 12 or more and 14 or less in the case of a di-long chain alkyl quaternary ammonium salt.

$R^{13}$ and $R^{14}$ are each independently a hydrogen atom or an alkyl group having 1 or more and 4 or less carbon atoms, and preferably a methyl group.

Examples of the mono-long-chain alkyl quaternary ammonium salt represented by the general formula (2) include monoalkyltrimethylammonium chlorides, such as octyltrimethylammonium chloride, decyltrimethylammonium chloride, dodecyltrimethylammonium chloride (lauryltrimethylammonium chloride), tetradecyltrimethylammonium chloride, hexadecyltrimethylammonium chloride, stearyltrimethylammonium chloride, cetyltrimethylammonium chloride, and behenyltrimethylammonium chloride.

Examples of the di-long chain alkyl quaternary ammonium salt represented by the general formula (2) include a dialkyl(C12-18)dimethylammonium chloride ("QUARTAMIN D-2345P", manufactured by Kao Corporation).

Examples of other cationic surfactant than the mono- or di-long chain alkyl quaternary ammonium salt represented by the general formula (2) include benzalkonium chlorides, such as octylbenzyldimethylammonium chloride; alkylpyridinium salts, such as cetylpyridinium chloride; and diethanolamine monoalkyl esters, triethanolamine monoalkyl esters, and triethanolamine dialkyl esters, or salts thereof.

Among those mentioned above, from the viewpoint of improvement in hair dyeing properties, in the compounds represented by the general formula (2), mono-long chain alkyl quaternary ammonium salts are preferred as the component (B); monoalkyltrimethylammonium chlorides are more preferred; at least one selected from the group consisting of

octyltrimethylammonium chloride, decyltrimethylammonium chloride, dodecyltrimethylammonium chloride (lauryltrimethylammonium chloride), tetradecyltrimethylammonium chloride, and hexadecyltrimethylammonium chloride is still more preferred; at least one selected from the group consisting of decyltrimethylammonium chloride, dodecyltrimethylammonium chloride, and tetradecyltrimethylammonium chloride is yet still more preferred; at least one selected from the group consisting of dodecyltrimethylammonium chloride and tetradecyltrimethylammonium chloride is even yet still more preferred; and dodecyltrimethylammonium chloride is even still more preferred.

From the viewpoint of improving the hair dyeing properties, the content of the component (B) in the composition of the present invention is 0.25 equivalents or more, preferably 0.3 equivalents or more, more preferably 0.4 equivalents or more, and still more preferably 0.5 equivalents or more relative to the component (A), and from the viewpoint of suppression of a lowering of touch to the hair and economy, it is less than 2 equivalents, preferably 1.5 equivalents or less, more preferably 1.2 equivalents or less, and still more preferably 1 equivalent or less relative to the component (A). A specific range of the content of the component (B) in the composition of the present invention is 0.25 equivalents or more and less than 2 equivalents, preferably 0.3 to 1.5 equivalents, more preferably 0.4 to 1.5 equivalents, still more preferably 0.4 to 1.2 equivalents, yet still more preferably 0.5 to 1.2 equivalents, and even yet still more preferably 0.5 to 1 equivalent relative to the component (A).

<Component (C): Alkaline Agent>

It is preferred that the composition of the present invention further contains an alkaline agent as a component (C). The alkaline agent has not only an action to swell the hair, thereby opening the cuticle and penetrating the component (A) and the like into the interior of the hair, but also an action to promote a polymerization reaction of the component (A) in the hair, thereby improving the hair dyeing properties. As the component (C), any material can be used without particular restrictions so long as it is an alkaline agent that is used for usual hair dyes.

Examples of the component (C) include ammonia; alkanolamines, such as mono-, di-, or tri-methanolamine and mono-, di-, or tri-ethanolamine; alkylamines, such as methylamine, dimethylamine, ethylamine, diethylamine, N-methylethylamine, propylamine, and butylamine; aralkylamines, such as benzylamine; basic amino acids, such as arginine, lysine, and histidine; and inorganic alkaline compounds, such as sodium hydroxide, potassium hydroxide, sodium carbonate, and potassium carbonate, and one or more of these materials can be used. The carbon number of the alkanolamine, alkylamine, or aralkylamine is preferably 10 or less, and more preferably 8 or less from the viewpoint of water solubility.

From the viewpoint of penetrating the component (A) into the interior of the hair, the component (C) preferably contains one or more selected from ammonia and an alkanolamine, more preferably contains a monoalkanolamine, and still more preferably contains monoethanolamine.

In the case where the component (C) contains one or more selected from ammonia and an alkanolamine, the content thereof in the component (C) is preferably 50% by mass or more, more preferably 70% by mass or more, still more preferably 80% by mass or more, and yet still more preferably 90% by mass or more, and it is 100% by mass or less.

In the case where the component (C) contains monoethanolamine, the content thereof in the component (C) is preferably 50% by mass or more, more preferably 70% by mass or more, still more preferably 80% by mass or more, and yet still more preferably 90% by mass or more, and it is 100% by mass or less.

From the viewpoint of not only penetrating the component (A) and the like into the interior of the hair but also promoting a polymerization reaction of the component (A) in the hair, thereby improving the hair dyeing properties, the content of the component (C) in the composition is preferably 0.01% by mass or more, more preferably 0.1% by mass or more, still more preferably 2% by mass or more, yet still more preferably 3% by mass or more, even yet still more preferably 3.5% by mass or more, even still more preferably 5% by mass or more, and even still more further preferably 7% by mass or more, and from the viewpoint of suppressing irritation, it is preferably 20% by mass or less, more preferably 15% by mass or less, still more preferably 12% by mass or less, and yet still more preferably 10% by mass or less. A specific range of the content of the component (C) in the composition is preferably 0.01 to 20% by mass, more preferably 0.1 to 15% by mass, still more preferably 2 to 12% by mass, yet still more preferably 2 to 10% by mass, even yet still more preferably 3 to 10% by mass, even still more preferably 3.5 to 10% by mass, even still more further preferably 5 to 10% by mass, and even yet still more further preferably 7 to 10% by mass.

[Aqueous Medium]

The composition of the present invention typically contains an aqueous medium. Examples of the aqueous medium include water; a lower alcohol, such as ethanol and isopropyl alcohol; and a low-molecular diol or triol having 6 or less carbon atoms, such as 1,3-butylene glycol, glycerin, ethylene glycol, and propylene glycol. The aqueous medium is preferably at least one selected from the group consisting of water and a lower alcohol, and it is preferred that the aqueous medium contains at least water.

Although the content of the aqueous medium in the composition can be appropriately selected according to the dosage form of the composition, it is typically in a range of 1 to 99.5% by mass. The content of the aqueous medium in the composition may be a remainder of all of the active components in the composition.

[Other Components]

The composition of the present invention may appropriately contain, in addition to the aforementioned components, a component which is usually used for hair cosmetics or hair dyes, within a range where the purpose of the present invention is not impaired. Examples of the foregoing component include a dyeing agent other than the component (A), an antioxidant, an anionic surfactant, a nonionic surfactant, an ampholytic surfactant, a polymer, an oil, a pH adjustor, an anti-dandruff agent, a disinfectant, an antiinflammatory agent, an antiseptic, a chelating agent, a humectant, a pearlescent agent, a ceramide, a fragrance, and an ultraviolet absorber.

Although the composition of the present invention may contain a polyoxyethylene addition type nonionic surfactant as the nonionic surfactant, its content is preferably low from the viewpoint of hair dyeing properties. In the composition of the present invention, the content of the polyoxyethylene addition type nonionic surfactant is preferably less than 1.0, more preferably 0.8 or less, and still more preferably 0.5 or less in terms of a mass ratio relative to the component (A) [(polyoxyethylene addition type nonionic surfactant)/(component (A))]. In addition, a lower limit value thereof is 0.

Although the composition of the present invention may contain ascorbic acid as the antioxidant, its content is preferably low. From the viewpoint of simplicity of the hair dyeing treatment, the content of ascorbic acid in the composition of the present invention is preferably less than 0.1% by mass, more preferably 0.08% by mass or less, still more preferably 0.05% by mass or less, yet still more preferably less than 0.01% by mass, and even yet still more preferably 0.008% by mass or less. In addition, a lower limit value thereof is 0% by mass.

A production method of the composition of the present invention is not particularly limited. For example, the composition of the present invention can be produced by blending the component (A), the component (B), and other components which are used as the need arises by a method described in the section of Examples and mixing the blend by using a known stirring device or the like. After mixing, the pH adjustment may be performed as the need arises.

<pH>

From the viewpoint of not only penetrating the component (A) into the interior of the hair but also promoting a polymerization reaction of the component (A) in the hair, thereby improving the hair dyeing properties, the pH of the composition of the present invention is preferably 8.0 or more, more preferably 8.5 or more, still more preferably 9.0 or more, and yet still more preferably 9.5 or more. This is because that the component (A) that is the melanin precursor readily reacts with oxygen in air under a basic condition, thereby causing polymerization. From the viewpoint of improving the hair dyeing properties and suppressing a damage on the hair, the pH is preferably 11.0 or less, and more preferably 10.5 or less. A specific range of the pH of the composition of the present invention is preferably 8.0 to 11.0, more preferably 8.5 to 11.0, still more preferably 9.0 to 11.0, and yet still more preferably 9.5 to 10.5.

The aforementioned pH is a measured value at 25°C, and specifically, it can be measured by a method described in the section of Examples.

[Dyeing Method of Hair]

The present invention further provides a dyeing method of hair including a step of applying the composition of the present invention on hair. In the step of applying the foregoing composition on hair, for example, the composition of the present invention is coated on the hair in a dry state or a wet state and then allowed to stand for 1 to 30 minutes, and more preferably 3 to 20 minutes for the purpose of penetrating the component (A) in the composition into the interior of the hair, followed by rinsing away with water.

The amount of the composition to be coated on the hair is preferably 0.05 or more, more preferably 0.1 or more, and still more preferably 0.25 or more, and it is preferably 1.5 or less, more preferably 1.25 or less, in terms of a bath ratio relative to the mass of the hair [(mass of composition)/(mass of hair)]. The hair which is an object to the treatment may be at least a part of hair.

Although the temperature in the aforementioned step is not particularly restricted, the step is performed at a temperature of preferably 40°C or lower, and more preferably 35°C or lower.

In accordance with the dyeing method of hair of the present invention, sufficient hair dyeing properties are obtained even with a one-time hair dyeing treatment. For example, in the case of dyeing gray hair, a color difference of hair ($\Delta E$ value) before and after the hair dyeing treatment as calculated according to the following equation is preferably 25 or more, and more preferably 30 or more.

$$\Delta E=[(L_1^*-L_0^*)^2+(a_1^*-a_0^*)^2+(b_1^*-b_0^*)^2]^{\frac{1}{2}}$$

$L_0^*$, $a_0^*$, $b_0^*$: colorimetric value before hair dyeing treatment
$L_1^*$, $a_1^*$, $b_1^*$: colorimetric value after one-time hair dyeing treatment

The $L_0^*$, $a_0^*$, $b_0^*$ and the $L_1^*$, $a_1^*$, $b_1^*$ are each an average value of values measured at 6 points by a C light source by using a spectrocolorimeter, and specifically, it can be measured by a method described in the section of Examples.

Regarding the aforementioned embodiments, the present invention discloses the following.

<1> A composition containing

(A) a compound represented by the following general formula (1) or a salt thereof:

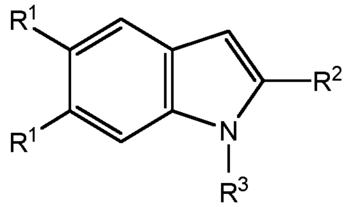

(1)

wherein two $R^1$'s each independently represent a hydroxy group or an acetoxy group; $R^2$ represents a hydrogen atom or -COOR (R is a hydrogen atom, a methyl group, or an ethyl group); and $R^3$ is a hydrogen atom, an acetyl group, or a methyl or ethyl group; and
(B) a cationic surfactant,
wherein,
the content of the component (B) is 0.25 equivalents or more and less than 2 equivalents relative to the component (A).

<2> The composition as set forth in <1>, wherein the component (A) is at least one selected from the group consisting of 5,6-dihydroxyindole, 5,6-dihydroxyindole-2-carboxylic acid, and salts thereof.
<3> The composition as set forth in <2>, wherein the component (A) contains 5,6-dihydroxyindole and 5,6-dihydroxyindole-2-carboxylic acid.
<4> The composition as set forth in <3>, wherein a molar ratio of 5,6-dihydroxyindole and 5,6-dihydroxyindole-2-carboxylic acid is in a range of 50/50 to 99.9/0.1.
<5> The composition as set forth in any one of <1> to <4>, wherein the content of the component (A) is 0.05 to 5% by mass.
<6> The composition as set forth in <5>, wherein the content of the component (A) is 0.2 to 2% by mass.
<7> The composition as set forth in any one of <1> to <6>, wherein the component (B) is at least one selected from the group consisting of primary to tertiary amine compounds having at least one hydrocarbon group having 8 or more and 22 or less carbon atoms, or salts thereof, quaternary ammonium salts, and pyridinium salts.
<8> The composition as set forth in any one of <1> to <7>, wherein the component (B) is a quaternary ammonium group having at least one hydrocarbon group having 8 or more and 22 or less carbon atoms and having the total carbon number of 36 or less.
<9> The composition as set forth in <8>, wherein the quaternary ammonium salt is a mono- or di-long chain alkyl quaternary ammonium salt represented by the following general formula (2):

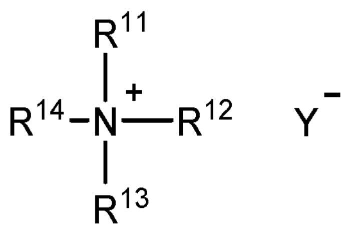

(2)

wherein $R^{11}$ represents a linear or branched alkyl group having 8 or more and 22 or less carbon atoms, or a group represented by $R^{15}CONH(CH_2)_m$-, $R^{15}$-O-$(CH_2)_m$-, or $R^{15}COO(CH_2)_m$- ($R^{15}$ represents a linear or branched alkyl group having 8 or more and 22 or less carbon atoms, and m represents a number of 1 or more and 4 or less); $R^{12}$ represents a linear or branched alkyl group having 1 or more and 22 or less carbon atoms, or a group represented by the foregoing $R^{15}CONH(CH_2)_m$-, $R^{15}$-O-$(CH_2)_m$-, or $R^{15}COO(CH_2)_m$-; $R^{13}$ and $R^{14}$ each independently represent an alkyl group having 1 or more and 4 or less carbon atoms; and $Y^-$ represents a chloride ion, a bromide ion, or a methosulfate ion.

<10> The composition as set forth in <9>, wherein the carbon number of the linear or branched alkyl group in $R^{11}$ and $R^{15}$ in the generation formula (2) is 10 or more and 14 or less.

<11> The composition as set forth in any one of <7> to <10>, wherein the total carbon number of the component (B) is 13 to 18.

<12> The composition as set forth in any one of <9> to <11>, wherein the quaternary ammonium salt is a mono-long chain alkyl quaternary ammonium salt.

<13> The composition as set forth in any one of <7> to <12>, wherein the component (B) is at least one selected from the group consisting of dodecyltrimethylammonium chloride and tetradecyltrimethylammonium chloride.

<14> The composition as set forth in any one of <1> to <13>, wherein the content of the component (B) is 0.5 to 1 equivalent relative to the component (A).

<15> The composition as set forth in any one of <1> to <14>, further containing an alkaline agent as a component (C).

<16> The composition as set forth in <15>, wherein the component (C) contains one or more selected from ammonia and an alkanolamine.

<17> The composition as set forth in <16>, wherein the component (C) contains one or more selected from ammonia and an alkanolamine, and the content thereof in the component (C) is 50 to 100% by mass.

<18> The composition as set forth in any one of <15> to <17>, wherein the component (C) contains monoethanolamine.

<19> The composition as set forth in <18>, wherein the content of monoethanolamine in the component (C) is 50 to 100% by mass.

<20> The composition as set forth in any one of <15> to <19>, wherein the content of the component (C) is 0.01 to 20% by mass.

<21> The composition as set forth in any one of <15> to <20>, wherein the content of the component (C) is 2 to 10% by mass, preferably 3 to 10% by mass, more preferably 3.5 to 10% by mass, still more preferably 5 to 10% by mass, and yet still more preferably 7 to 10% by mass.

<22> The composition as set forth in any one of <1> to <21>, wherein the content of a polyoxyethylene addition type nonionic surfactant is less than 1.0 in terms of a mass ratio relative to the component (A) [(polyoxyethylene addition type nonionic surfactant)/(component (A))].

<23> The composition as set forth in any one of <1> to <22>, wherein the pH is 8.0 or more and 11.0 or less.

<24> The composition as set forth in <1> or <2>, containing

(A) at least one selected from the group consisting of 5,6-dihydroxyindole, 5,6-dihydroxyindole-2-carboxylic acid, and salts thereof; and
(B) a quaternary ammonium salt having at least one hydrocarbon group having 8 or more and 22 or less carbon atoms and having the total carbon number of 36 or less, wherein
the content of the component (A) is 0.05 to 5% by mass, and the content of the component (B) is 0.25 equivalents or more and less than 2 equivalents relative to the component (A).

<25> The composition as set forth in <1> or <2>, containing

(A) 5,6-dihydroxyindole and 5,6-dihydroxyindole-2-carboxylic acid; and
(B) a quaternary ammonium salt having at least one hydrocarbon group having 8 or more and 22 or less carbon atoms and having the total carbon number of 36 or less,
wherein,

a molar ratio of 5,6-dihydroxyindole and 5,6-dihydroxyindole-2-carboxylic acid is in a range of 50/50 to 99.9/0.1, and
the content of the component (A) is 0.05 to 5% by mass, and the content of the component (B) is 0.25 equivalents or more and less than 2 equivalents relative to the component (A).

<26> The composition as set forth in <1> or <2>, containing

(A) 5,6-dihydroxyindole and 5,6-dihydroxyindole-2-carboxylic acid; and
(B) a mono- or di-long chain alkyl quaternary ammonium salt represented by the following general formula (2):

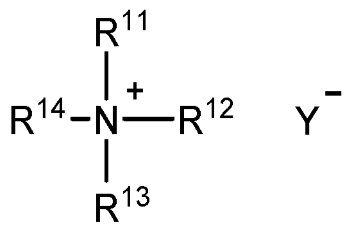

(2)

wherein $R^{11}$ represents a linear or branched alkyl group having 8 or more and 22 or less carbon atoms, or a group represented by $R^{15}CONH(CH_2)_m$-, $R^{15}$-O-$(CH_2)_m$-, or $R^{15}COO(CH_2)_m$- ($R^{15}$ represents a linear or branched alkyl group having 8 or more and 22 or less carbon atoms, and m represents a number of 1 or more and 4 or less); $R^{12}$ represents a linear or branched alkyl group having 1 or more and 22 or less carbon atoms, or a group represented by the foregoing $R^{15}CONH(CH_2)_m$-, $R^{15}$-O-$(CH_2)_m$-, or $R^{15}COO(CH_2)_m$-; $R^{13}$ and $R^{14}$ each independently represent an alkyl group having 1 or more and 4 or less carbon atoms; and $Y^-$ represents a chloride ion, a bromide ion, or a methosulfate ion, wherein,
the content of the component (A) is 0.2 to 2% by mass, and the content of the component (B) is 0.25 equivalents or more and less than 2 equivalents relative to the component (A)

<27> The composition as set forth in <26>, containing

(A) 5,6-dihydroxyindole and 5,6-dihydroxyindole-2-carboxylic acid; and
(B) at least one cationic surfactant selected from the group consisting of dodecyltrimethylammonium chloride and tetradecyltrimethylammonium chloride, wherein,
the content of the component (A) is 0.2 to 2% by mass, and the content of the component (B) is 0.25 equivalents or more and less than 2 equivalents relative to the component (A).

<28> The composition as set forth in <26> or <27>, containing

(A) 5,6-dihydroxyindole and 5,6-dihydroxyindole-2-carboxylic acid; and
(B) at least one cationic surfactant selected from the group consisting of dodecyltrimethylammonium chloride and tetradecyltrimethylammonium chloride, wherein,
the content of the component (A) is 0.2 to 2% by mass, and the content of the component (B) is 0.5 to 1 equivalent relative to the component (A).

<29> The composition as set forth in any one of <26> to <28>, containing

(A) 5,6-dihydroxyindole and 5,6-dihydroxyindole-2-carboxylic acid; and
(B) at least one cationic surfactant selected from the group consisting of dodecyltrimethylammonium chloride and tetradecyltrimethylammonium chloride, wherein,
the content of the component (A) is 0.2 to 2% by mass; the content of the component (B) is 0.5 to 1 equivalent relative to the component (A); and the content of a polyoxyethylene addition type nonionic surfactant is less than 1.0 in terms of a mass ratio relative to the component (A) [(polyoxyethylene addition type nonionic surfactant)/(component (A))].

<30> A composition containing

(a) a compound represented by the following general formula (1) or a salt thereof:

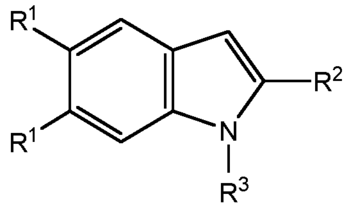

(1)

wherein two $R^1$'s each independently represent a hydroxy group or an acetoxy group; $R^2$ represents a hydrogen atom or -COOR (R is a hydrogen atom, a methyl group, or an ethyl group); and $R^3$ is a hydrogen atom, an acetyl group, or a methyl or ethyl group; and

(B) at least one cationic surfactant selected from the group consisting of dodecyltrimethylammonium chloride and tetradecyltrimethylammonium chloride, wherein,

the content of the component (A) is 0.2 to 2% by mass but not 0.29% by mass; the content of the component (B) is 0.25 equivalents or more and less than 2 equivalents relative to the component (A); and the content of a polyoxyethylene addition type nonionic surfactant is less than 1.0 in terms of a mass ratio relative to the component (A) [(polyoxyethylene addition type nonionic surfactant)/(component (A))].

<31> The composition as set forth in <30>, wherein the component (A) is 5,6-dihydroxyindole and 5,6-dihydroxyindole-2-carboxylic acid.

<32> The composition as set forth in <30> or <31>, wherein the content of the component (B) is 0.5 to 1 equivalent relative to the component (A).

<33> A composition containing

(a) a compound represented by the following general formula (1) or a salt thereof:

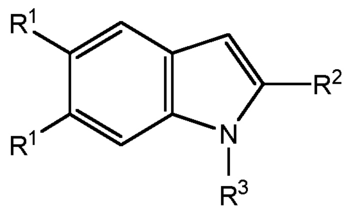

(1)

wherein two $R^1$'s each independently represent a hydroxy group or an acetoxy group; $R^2$ represents a hydrogen atom or -COOR (R is a hydrogen atom, a methyl group, or an ethyl group); and $R^3$ is a hydrogen atom, an acetyl group, or a methyl or ethyl group;

(B) at least one cationic surfactant selected from the group consisting of dodecyltrimethylammonium chloride and tetradecyltrimethylammonium chloride; and

(C) monoethanolamine,

wherein,

the content of the component (B) is 0.25 equivalents or more and less than 2 equivalents relative to the component (A); the content of the component (C) is 0.01 to 20% by mass but not 3.00% by mass; and the content of a polyoxyethylene addition type nonionic surfactant is less than 1.0 in terms of a mass ratio relative to the component (A) [(polyoxyethylene addition type nonionic surfactant)/(component (A))].

<34> The composition as set forth in <33>, wherein the component (A) is 5,6-dihydroxyindole and 5,6-dihydroxyindole-2-carboxylic acid.

<35> The composition as set forth in <33> or <34>, wherein the content of the component (B) is 0.5 to 1 equivalent relative to the component (A).

<36> The composition as set forth in any one of <33> to <35>, wherein the content of the component (C) is 2 to 10% by mass but not 3.00% by mass.

<37> A composition containing

(a) a compound represented by the following general formula (1) or a salt thereof:

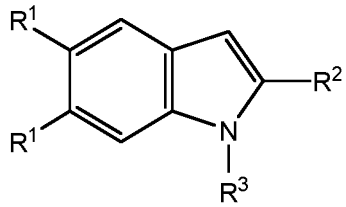

(1)

wherein two $R^1$'s each independently represent a hydroxy group or an acetoxy group; $R^2$ represents a hydrogen atom or -COOR (R is a hydrogen atom, a methyl group, or an ethyl group); and $R^3$ is a hydrogen atom, an acetyl group, or a methyl or ethyl group; and
(B) at least one cationic surfactant selected from the group consisting of dodecyltrimethylammonium chloride and tetradecyltrimethylammonium chloride, wherein,

the content of the component (B) is 0.25 equivalents or more and less than 2 equivalents relative to the component (A); and the content of a polyoxyethylene addition type nonionic surfactant is less than 1.0 in terms of a mass ratio relative to the component (A) [(polyoxyethylene addition type nonionic surfactant)/(component (A))], provided that the following hair cosmetic is excluded:

a hair cosmetic obtained by adding to an aqueous solution prepared by mixing
a part of ascorbic acid (ascorbic acid (JP), manufactured by Watanabe Chemical Co., Ltd.),
1.61% by mass of lauryltrimethylammonium chloride (QUARTAMIN 24P, manufactured by Kao Corporation, amount of active component: 27.5% by mass),
3.00% by mass of monoethanolamine (manufactured by Petronas Chemicals),
1.10% by mass of 75% phosphoric acid (food additive, 75% phosphoric acid, manufactured by Nippon Chemical Industrials Co., Ltd.), and
69.19% by mass of water,
ascorbic acid in a remainder amount such that the whole amount of ascorbic acid in the hair cosmetic is 0.10% by mass, and 25.00% by mass of a solution (5,6-dihydroxyindole: 1% by mass, 5,6-dihydroxyindole-2-carboxylic acid: 0.14% by mass, ethanol: 20% by mass, water: balance) as produced by the method described in Japanese Patent No. 5570161, the hair cosmetic having a pH at 25°C of 10.2.

<38> The composition as set forth in <37>, wherein the component (A) is 5,6-dihydroxyindole and 5,6-dihydroxyindole-2-carboxylic acid.
<39> The composition as set forth in <37> or <38>, wherein the content of the component (B) is 0.5 to 1 equivalent relative to the component (A).
<40> The composition as set forth in any one of <1> to <39>, which is a hair dye.
<41> A method for dyeing hair including a step of applying the composition as set forth in any one of <1> to <40> on hair.

Example

The present invention is hereunder described by reference to Examples, but it should be construed that the present invention is not limited to the scope of the Examples. In the present Examples, the respective measurements and evaluations were performed by the following methods.

[pH Measurement]

A pH at 25°C of the hair dye was measured with a pH meter (manufactured by HORIBA, Ltd., "D-71S").

[Evaluation of Hair Dyeing Properties]

On 1 g of a dried Chinese gray hair tress (available from Beaulax Co., Ltd., length: 10 cm, a trade name: Human Gray Hair (100%), trade No.: BM-W-A), the hair dye obtained in each of the Examples was coated in a bath ratio of 1/1 and then allowed to stand at 30°C for 15 minutes. The resultant was rinsed with running water for 30 seconds and then cleansed with a plain shampoo having the following composition for 15 seconds, followed by rinsing with running water for 15 seconds. This rinsing and cleansing operation was repeated two times. Subsequently, the resultant was subjected to a conditioner treatment with a plain conditioner having the following composition for 15 seconds, rinsed with running

water for 15 seconds, and then dried with cool air, thereby obtaining a tress after the one-time hair dyeing treatment. With respect to one tress before and after the hair dyeing treatment, the L*, a*, b* value was measured at 6 points by a C light source by using a spectrocolorimeter "CR-300", manufactured by Konica Minolta Japan, Inc., and an average value thereof was defined as a colorimetric value. The ΔE value was calculated according to the following equation, thereby evaluating the hair dyeing properties after the one-time hair dying treatment. It is meant that the larger the ΔE value, the more excellent the hair dyeing properties. In addition, when the ΔE value determined by the aforementioned evaluation method is 25 or more, it is judged that the sufficient hair dyeing properties are obtained by the one-time hair dyeing treatment.

$$\Delta E=[(L_1*-L_0*)^2+(a_1*-a_0*)^2+(b_1*-b_0*)^2]^{\frac{1}{2}}$$

$L_0$*, $a_0$*, $b_0$*: colorimetric value of tress before hair dyeing treatment
$L_1$*, $a_1$*, $b_1$*: colorimetric value of tress after one-time hair dyeing treatment

[Composition of Plain Shampoo]

| Component | (% by mass) |
|---|---|
| Sodium polyoxyethylene lauryl ether sulfate | 11.3 (*1) |
| Coconut oil fatty acid N-methylethanolamide (*2) | 3.0 |
| Citric acid | 0.2 |
| Methyl paraben | 0.3 |
| Purified water | Balance |
| Total | 100.0 |

*1: 42.0% by mass as EMAL E-27C (manufactured by Kao Corporation, active component: 27% by mass)
*2: AMINON C-11S (manufactured by Kao Corporation)

[Composition of Plain Conditioner]

| Component | (% by mass) |
|---|---|
| Distearyldimethylammonium chloride | 2.7 (*1) |
| Stearyltrimethylammonium chloride | 0.76 (*2) |
| Cetanol | 2.0 |
| Propylene glycol | 5.0 |
| Purified water | Balance |
| Total | 100.0 |

*1: 3.6% by mass as QUARTAMIN D86P (manufactured by Kao Corporation, active component: 75% by mass)
*2: 2.7% by mass as QUARTAMIN 86W (manufactured by Kao Corporation, active component: 28% by mass)

Examples 1 to 2 and Comparative Examples 1 to 3 (Production and Evaluation of Hair Dye)

Monoethanolamine as the component (C) and deionized water were blended according to a composition shown in Table 1 to prepare a solution, and nitrogen was blown into this solution in a nitrogen atmosphere for 1 hour. Subsequently, the component (A) and the component (B) in each of Examples 1 to 2 and Comparative Examples 2 to 3 were blended in a composition shown in Table 1, to obtain a hair dye. The blending amounts (% by mass) described in Table 1 are all the amounts of the active components except for ethanol.
With respect to the obtained hair dyes, the evaluation of hair dyeing properties was carried out by the aforementioned method. The results are shown in Table 1.

Table 1

| | | | | | Example 1 | Example 2 | Comparative Example 1 | Comparative Example 2 | Comparative Example 3 |
|---|---|---|---|---|---|---|---|---|---|
| Composition | Blending amount [% by mass] | (A) | 5,6-Dihydroxyindole | *1 | 0.22 | 0.22 | 0.22 | 0.22 | 0.22 |
| | | | 5,6-Dihydroxyindole-2-carboxylic acid | | 0.03 | 0.03 | 0.03 | 0.03 | 0.03 |
| | | (B) | Dodecyltrimethylammonium chloride | *2 | 0.22 | 0.44 | 0 | 0.88 | 0.066 |
| | | (C) | Monoethanolamine | *3 | 7 | 10 | 10 | 10 | 10 |
| | | Others | 95% by volume ethanol | *4 | 6.25 | 6.25 | 6.25 | 6.25 | 6.25 |
| | | | Deionized water | | Balance | Balance | Balance | Balance | Balance |
| | | Total | | | 100 | 100 | 100 | 100 | 100 |
| | pH | | | | 10 | 10 | 10 | 10 | 10 |
| | Equivalents of (B) relative to (A) | | | | 0.50 | 1.0 | 0 | 2.0 | 0.15 |
| Evaluation results | | Evaluation of hair dyeing properties (ΔE) | | | 31 | 33 | 23 | 24 | 22.4 |

*1: A solution (5,6-dihydroxyindole: 1% by mass, 5,6-dihydroxyindole-2-carboxylic acid: 0.14% by mass [a molar ratio of 5,6-dihydroxyindole and 5,6-dihydroxyindole-2-carboxylic acid is 90/10], ethanol: 20% by mass, water: balance) as produced by the method described in Japanese Patent No. 5570161 was blended in the amounts of the active components described in Table 1.
*2: Manufactured by Tokyo Chemical Industry Co., Ltd.
*3: "ETHANOLAMINE", manufactured by FUJIFILM Wako Pure Chemical Corporation
*4: Manufactured by FUJIFILM Wako Pure Chemical Corporation

From Table 1, it was noted that the hair dyes of Examples 1 and 2 provided more excellent hair dyeing properties than the hair dye of Comparative Example 1 containing the component (A) of the same kind and amount but not containing the component (B) and the hair dyes of Comparative Examples 2 and 3 in which the content of the component (B) falls outside the scope of the present invention.

Industrial Applicability

When the composition of the present invention is applied on hair, sufficient hair dyeing properties are obtained even with a one-time hair dyeing treatment. The composition of the present invention is, for example, useful as a hair dye.

## Claims

1. A composition comprising (A) a compound represented by the following general formula (1) or a salt thereof:

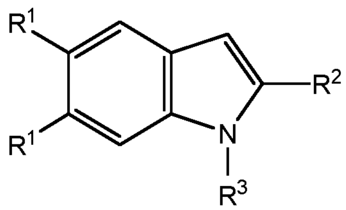

(1)

wherein two $R^1$'s each independently represent a hydroxy group or an acetoxy group; $R^2$ represents a hydrogen atom or -COOR (R is a hydrogen atom, a methyl group, or an ethyl group); and $R^3$ is a hydrogen atom, an acetyl group, or a methyl or ethyl group; and
(B) a cationic surfactant,
wherein,
the content of the component (B) is 0.25 equivalents or more and less than 2 equivalents relative to the component (A).

2. The composition according to claim 1, wherein the component (A) is at least one selected from the group consisting of 5,6-dihydroxyindole, 5,6-dihydroxyindole-2-carboxylic acid, and salts thereof.

3. The composition according to claim 1 or 2, wherein the component (B) is a quaternary ammonium salt having at least one hydrocarbon group having 8 or more and 22 or less carbon atoms and having the total carbon number of 36 or less.

4. The composition according to any one of claims 1 to 3, wherein the pH is 8.0 or more and 11.0 or less.

5. The composition according to any one of claims 1 to 4, further comprising an alkaline agent as a component (C).

6. The composition according to any one of claims 1 to 5, which is a hair dye.

7. A method for dyeing hair comprising a step of applying the composition according to any one of claims 1 to 6 on hair.

**INTERNATIONAL SEARCH REPORT**

International application No.
PCT/JP2019/049976

A. CLASSIFICATION OF SUBJECT MATTER
A61Q 5/06(2006.01)i; A61K 8/40(2006.01)i; A61K 8/41(2006.01)i; A61K 8/49(2006.01)i
FI: A61K8/49; A61Q5/06; A61K8/40; A61K8/41

According to International Patent Classification (IPC) or to both national classification and IPC

B. FIELDS SEARCHED

Minimum documentation searched (classification system followed by classification symbols)
A61Q5/06; A61K8/40; A61K8/41; A61K8/49

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | JP 2001-181159 A (LION CORP.) 03.07.2001 (2001-07-03) example 5 | 1-7 |
| Y | paragraphs [0006]-[0008] | 2-7 |
| X | JP 2002-53436 A (LION CORP.) 19.02.2002 (2002-02-19) examples 25, 26 | 1-7 |
| Y | paragraphs [0052]-[0055] | 2-7 |
| X | JP 2018-070503 A (REAL CHEMICAL CO., LTD.) 10.05.2018 (2018-05-10) example 12, paragraph [0031] | 1-3,5-7 |
| Y | claim 2, paragraphs [0001]-[0009], [0018] | 2-7 |
| X | JP 2001-10938 A (LION CORP.) 16.01.2001 (2001-01-16) example 16 | 1-3,5-7 |
| Y | paragraphs [0012]-[0017], [0032] | 2-7 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
06 March 2020 (06.03.2020)

Date of mailing of the international search report
24 March 2020 (24.03.2020)

Name and mailing address of the ISA/
Japan Patent Office
3-4-3, Kasumigaseki, Chiyoda-ku,
Tokyo 100-8915, Japan

Authorized officer

Telephone No.

Form PCT/ISA/210 (second sheet) (January 2015)

**INTERNATIONAL SEARCH REPORT**

International application No.
PCT/JP2019/049976

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2007-326807 A (KAO CORP.) 20.12.2007 (2007-12-20) claim 3, paragraph [0016] | 2-7 |
| A | JP 2016-98228 A (NISSIN SUGAR CO., LTD.) 30.05.2016 (2016-05-30) paragraph [0044] | 1-7 |
| A | JP 2014-24766 A (REAL CHEMICAL CO., LTD.) 06.02.2014 (2014-02-06) | 1-7 |
| A | JP 2007-326803 A (KAO CORP.) 20.12.2007 (2007-12-20) | 1-7 |
| A | WO 2008/149535 A1 (KAO CORP.) 11.12.2008 (2008-12-11) | 1-7 |
| A | JP 2002-47144 A (KAO CORP.) 12.02.2002 (2002-02-12) | 1-7 |

Form PCT/ISA/210 (continuation of second sheet) (January 2015)

**INTERNATIONAL SEARCH REPORT**
Information on patent family members

International application No.
PCT/JP2019/049976

| Patent Documents referred in the Report | Publication Date | Patent Family | Publication Date |
|---|---|---|---|
| JP 2001-181159 A | 03 Jul. 2001 | (Family: none) | |
| JP 2002-53436 A | 19 Feb. 2002 | (Family: none) | |
| JP 2018-070503 A | 10 May 2018 | (Family: none) | |
| JP 2001-10938 A | 16 Jan. 2001 | WO 2001/000144 A2 example 16, page 5, line 10 to page 6, line 4, page 48, lines 13-14<br>EP 1189576 A2<br>AU 5567700 A | |
| JP 2007-326807 A | 20 Dec. 2007 | US 2010/0170048 A1 paragraph [0013], claim 4<br>WO 2007/141920 A1<br>EP 2030606 A1<br>KR 10-2009-0016570 A<br>CN 101460136 A<br>TW 200303370 A | |
| JP 2016-98228 A | 30 May 2016 | (Family: none) | |
| JP 2014-24766 A | 06 Feb 2014 | (Family: none) | |
| JP 2007-326803 A | 20 Dec. 2007 | US 2010/0170048 A1<br>WO 2007/141920 A1<br>EP 2030606 A1<br>KR 10-2009-0016570 A<br>CN 101460136 A<br>TW 200303370 A | |
| WO 2008/149535 A1 | 11 Dec. 2008 | US 2010/0154135 A1<br>EP 2151232 A1 | |
| JP 2002-47144 A | 12 Feb. 2002 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2007326810 A
- JP 2009137877 A
- JP 5570161 B